# FASCICULE DE BREVET EUROPEEN

(11) **EP 1 059 952 B1**
(45) Date de publication et mention de la délivrance du brevet: **22.10.2003**
(21) Numéro de dépôt: 99907590.6
(22) Date de dépôt: 03.03.1999
(51) Int. Cl.: A61M 15/00

(54) **DISPOSITIF DE DISTRIBUTION DE PRODUIT FLUIDE DECLENCHE PAR L'INHALATION**
INHALATIONSBETÄTIGTE ABGABEVORRICHTUNG FÜR EIN FLÜSSIGES PRODUKT
DISPENSING DEVICE FOR FLUID PRODUCT ACTIVATED BY INHALING

(30) Priorité: 04.03.1998 FR 9802590
(43) Date de publication de la demande: 20.12.2000
(73) Titulaire: TEBRO, 1118 Luxembourg (LU)
(72) Inventeur: STRADELLA, Giuseppe, I-16032 Camogli (IT)
(74) Mandataire: CAPRI SARL
(86) Numéro de dépôt international: EP9901368
(87) Numéro de publication internationale: WO99044662

(56) Documents cités:
- EP-A- 0 414 536
- WO-A-98/52634
- GB-A- 2 292 891
- US-A- 3 157 179
- US-A- 5 060 643
- US-A- 5 507 281
- US-A- 5 546 932

## Description

La présente invention concerne un dispositif de distribution de produit fluide déclenché par l'inhalation, et plus particulièrement un dispositif du type aérosol déclenché par l'inhalation.

Les dispositifs déclenchés par l'inhalation (désignés généralement par le terme B.A.I.) sont bien connus de l'état de la technique voir par exemple le document EP-0 414 536. L'avantage principal de ce type de dispositif est que la distribution du produit est synchronisée avec l'inhalation du patient, pour garantir une bonne distribution du produit. Ainsi, dans le domaine des dispositifs aérosol, c'est-à-dire ceux dans lesquels le produits est distribué à l'aide d'un gaz propulseur, de nombreux types de dispositifs de déclenchement par l'inhalation ont été proposés. Ces dispositifs présentent toutefois tous l'inconvénient de comporter un grand nombre de pièces, c'est-à-dire qu'ils sont compliqués et coûteux à fabriquer et à assembler, ce qui est évidemment désavantageux.

La présente invention a pour but de fournir un dispositif de distribution de produit fluide déclenché par l'inhalation qui comporte un minimum de pièces constitutives, et qui est donc simple et peu coûteux à fabriquer et à assembler.

La présente invention a aussi pour but de fournir un dispositif de distribution de produit fluide déclenché par l'inhalation, qui fonctionne de manière fiable et qui ne nécessite pas une aspiration importante pour déclencher la distribution du produit.

La présente invention a donc pour objet un dispositif de distribution de produit fluide déclenché par l'inhalation, comportant un réservoir de produit contenant un gaz propulseur, une pompe ou valve doseuse pour distribuer sélectivement le produit, et un dispositif de déclenchement commandé par l'inhalation, ledit dispositif de déclenchement comportant :
- un élément de commande mobile entre une position de repos et une position de commande, ledit élément de commande étant déplacé par l'utilisateur,
- un organe d'actionnement, mobile entre une position de non-actionnement, dans laquelle la pompe ou valve doseuse n'est pas actionnée, et une position d'actionnement, dans laquelle la pompe ou valve doseuse est actionnée, ledit organe d'actionnement étant sollicité vers sa position d'actionnement lorsque l'élément de commande est dans sa position de commande,
- un élément de retenue, mobile entre une position de retenue, dans laquelle il coopère avec l'organe d'actionnement pour le retenir dans sa position de non-actionnement, et une position de libération, dans laquelle ledit organe d'actionnement se déplace dans sa position d'actionnement, et
- un élément de came, mobile entre une position de stockage, dans laquelle ledit élément de retenue est bloqué en position de retenue, et une position d'inhalation, dans laquelle ledit élément de retenue se déplace dans sa position de libération, ledit élément de came étant sollicité vers sa position de stockage et étant déplacé dans sa position d'inhalation lors de l'inhalation par l'utilisateur du dispositif.

De préférence, le dispositif comporte en outre un organe de blocage coopérant d'une part avec l'élément de retenue et d'autre part avec l'élément de came, ledit organe de blocage étant mobile entre une position de blocage, dans laquelle il bloque ledit élément de retenue en position de retenue, et une position de déblocage, dans laquelle ledit élément de retenue se déplace dans sa position de libération, ledit organe de blocage étant en position de blocage lorsque ledit élément de came est dans sa position de stockage, et en position de déblocage, lorsque ledit élément de came est dans sa position d'inhalation.

Avantageusement, le déplacement de l'élément de commande vers sa position de commande comprime un ressort qui sollicite l'organe d'actionnement vers sa position d'actionnement.

Avantageusement, le ressort est disposé entre l'élément de commande et l'organe d'actionnement.

De préférence, le dispositif comporte un boîtier recevant le réservoir, la pompe ou valve doseuse et le dispositif de déclenchement, le boîtier comportant en outre un orifice de distribution, tel qu'un embout buccal, à travers lequel l'utilisateur inhale, et une ouverture, obturée par l'élément de came en position de stockage.

Avantageusement, ledit élément de came est monté pivotant dans le boîtier, l'inhalation de l'utilisateur créant une dépression dans le boîtier qui fait pivoter l'élément de came vers sa position d'inhalation, dans laquelle il n'obture plus l'ouverture du boîtier.

Avantageusement, l'élément de commande est monté pivotant sur le boîtier et obture en position de repos l'orifice de distribution du dispositif.

De préférence, l'élément de retenue est élastiquement déformable entre sa position de retenue et sa position de libération, l'élément de retenue étant sollicité vers sa position de libération par l'organe d'actionnement lorsque l'élément de commande est dans sa position de commande.

Avantageusement, l'élément de retenue est logé dans une rainure de l'organe d'actionnement, ladite rainure ayant une paroi latérale oblique, qui, lorsque l'organe d'actionnement est sollicité vers sa position d'actionnement, exerce une force sur l'élément de retenue pour le solliciter vers sa position de libération.

Avantageusement, l'organe de blocage est un manchon cylindrique monté coulissant autour de l'élément de retenue, ledit manchon comportant une première partie coopérant en position de blocage avec l'élément de retenue pour l'empêcher de se déplacer vers sa position de libération, et une seconde partie de plus grand diamètre coopérant en position de déblocage avec l'élément de retenue en lui permettant de se déplacer vers sa position de libération.

Avantageusement, la seconde partie du manchon est conique, de telle sorte que lorsque le manchon est ramené dans sa position de blocage, la partie conique du manchon coopère avec l'élément de retenue pour le solliciter vers sa position de retenue.

De préférence, le dispositif comporte en outre un élément de structure fixé au boîtier, l'élément de came et l'élément de commande étant montés pivotants sur ledit élément de structure.

Selon une première variante avantageuse, l'élément de retenue est un anneau fendu déformable.

Selon une seconde variante avantageuse, l'élément de retenue comporte au moins une patte flexible déformable solidaire dudit élément de structure.

Avantageusement, après distribution de la dose de produit par la pompe ou valve doseuse, l'élément de commande, lorsqu'il est ramené dans sa position de repos, ramène l'organe d'actionnement dans sa position de non-actionnement, de sorte que l'élément de retenue est ramené dans sa position de retenue, l'organe de blocage est ramené dans sa position de blocage et l'élément de came est ramené dans sa position de stockage.

D'autres caractéristiques et avantages de la présente invention apparaîtront plus clairement au cours de la description détaillée suivante donnée à titre d'exemple non limitatif en regard des dessins joints, sur lesquels :
- la figure 1 est une vue schématique en coupe d'un dispositif de distribution selon un mode de réalisation avantageux de la présente invention, en position de repos,
- la figure 2 est une vue schématique en coupe selon un autre plan de coupe représentant le dispositif de la figure 1, également en position de repos,
- la figure 3 est une vue similaire à celle de la figure 1, incorporant des variantes de réalisation, également en position de repos,
- la figure 4 est une vue similaire à celle de la figure 1, avant inhalation, et
- la figure 5 est une vue similaire à celle de la figure 4, après inhalation.

En référence aux dessins, le dispositif de distribution comporte un réservoir de produit 20, contenant le produit à distribuer et un gaz propulseur, et une pompe ou valve doseuse 30 montée sur ledit réservoir 20 pour distribuer sélectivement le produit. L'orifice de sortie de la pompe ou valve doseuse 30 est relié via un canal à un orifice de distribution 9, tel qu'un embout buccal, à travers lequel l'utilisateur inhale le produit distribué. Cet orifice de distribution peut bien entendu être réalisé d'une manière quelconque en fonction de l'application souhaitée du dispositif. La présente description sera faite en référence à un inhalateur comportant un embout buccal, mais il est clair que l'invention s'applique également à d'autres types d'utilisations, tels que par exemple un traitement endonasal.

Selon l'invention, le dispositif comporte un dispositif de déclenchement commandé par l'inhalation de l'utilisateur, qui est destiné à actionner la pompe ou valve doseuse 30 lorsque l'utilisateur inhale à travers l'embout buccal 9. Avantageusement, le réservoir 20, la pompe ou valve doseuse 30 et le dispositif de déclenchement par l'inhalation sont contenus dans un boîtier 1, qui incorpore avantageusement l'orifice de distribution 9, et sur lequel est monté un élément de commande 3 qui obture en position de repos l'orifice de distribution 9.

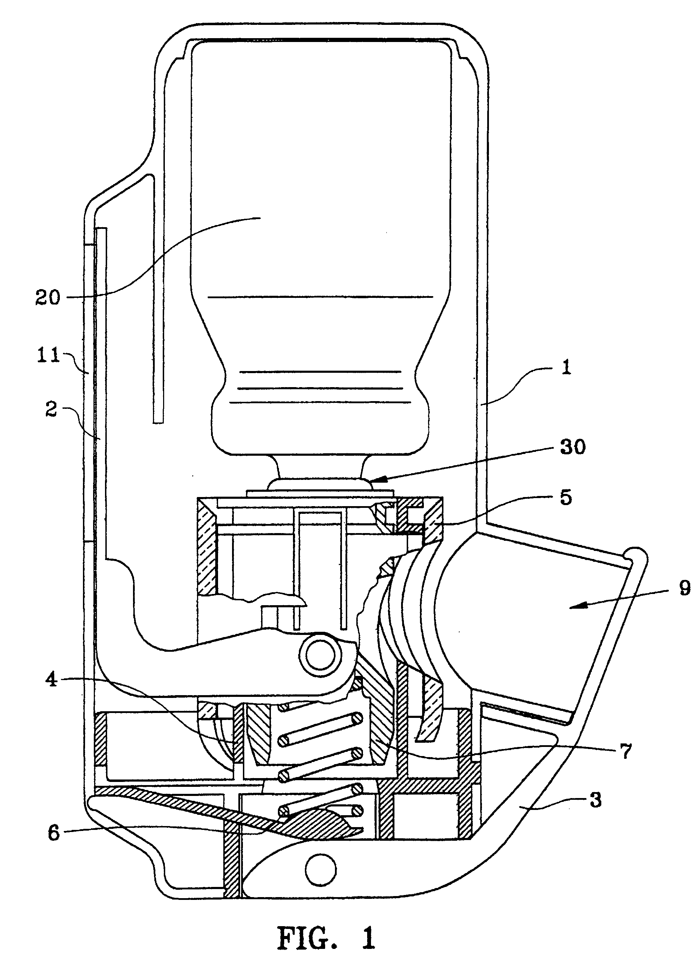

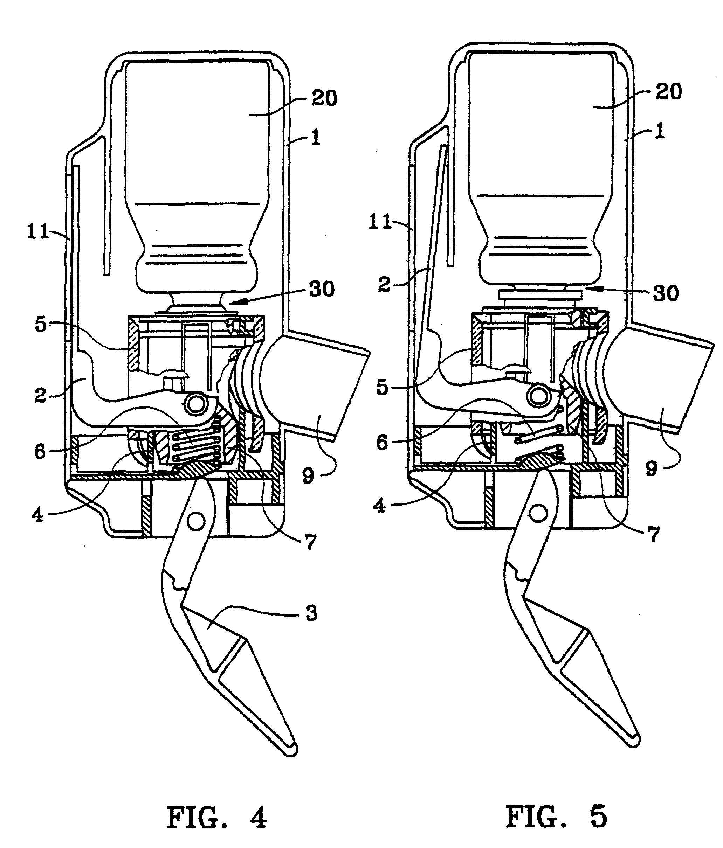

Selon l'invention, cet élément de commande 3 est mobile entre sa position de repos représentée sur les figures 1, 2 et 3, et une position de commande représentée sur les figures 4 et 5. Le déplacement de l'élément de commande 3 de sa position de repos dans sa position de commande a pour effet de précontraindre le dispositif de déclenchement, qui sera ensuite déclenché par l'inhalation, avec pour conséquence la distribution d'une dose de produit à travers l'orifice de distribution 9. Le dispositif de déclenchement par l'inhalation comporte à cet effet un élément de came 2 qui comporte une partie, telle qu'un volet, adaptée à coopérer avec une ouverture 11 prévue dans le boîtier 1 du dispositif. Cette ouverture 11 est de préférence réalisée dans le boîtier 1 à l'opposé de l'orifice de distribution à travers lequel l'utilisateur inhale, de sorte que lors de l'inhalation il se crée une dépression à l'intérieur du boîtier 1 provoquant le déplacement de l'élément de came 2 d'une position de stockage représentée sur les figures 1, 3 et 4, vers une position d'inhalation, représentée sur la figure 5. L'élément de came 2 est, au repos, sollicité vers sa position de stockage, mais cette sollicitation est suffisamment faible pour être surmontée même par une faible inhalation. Le dispositif de déclenchement par l'inhalation comporte en outre un organe d'actionnement 7 qui est mobile entre une position de non-actionnement, dans laquelle la pompe ou valve doseuse 30 n'est pas actionnée, telle que représenté sur les figures 1 à 4, et une position d'actionnement dans laquelle la pompe ou valve doseuse 30 est actionnée pour distribuer une dose de produit, comme représenté sur la figure 5. Lorsque l'élément de commande 3 est déplacé vers sa position de commande, l'organe d'actionnement 7 est sollicité en direction de sa position d'actionnement. Ceci est de préférence réalisé au moyen d'un ressort 6 qui est adapté à solliciter l'organe d'actionnement 7 vers sa position d'actionnement. Comme représenté sur les figures, le ressort 6 peut de préférence être disposé entre l'élément de commande 3 et l'organe d'actionnement 7. Bien entendu, on pourrait également prévoir ce ressort à un autre endroit du dispositif, par exemple agissant sur le réservoir 20 pour le solliciter en direction de l'organe d'actionnement 7.

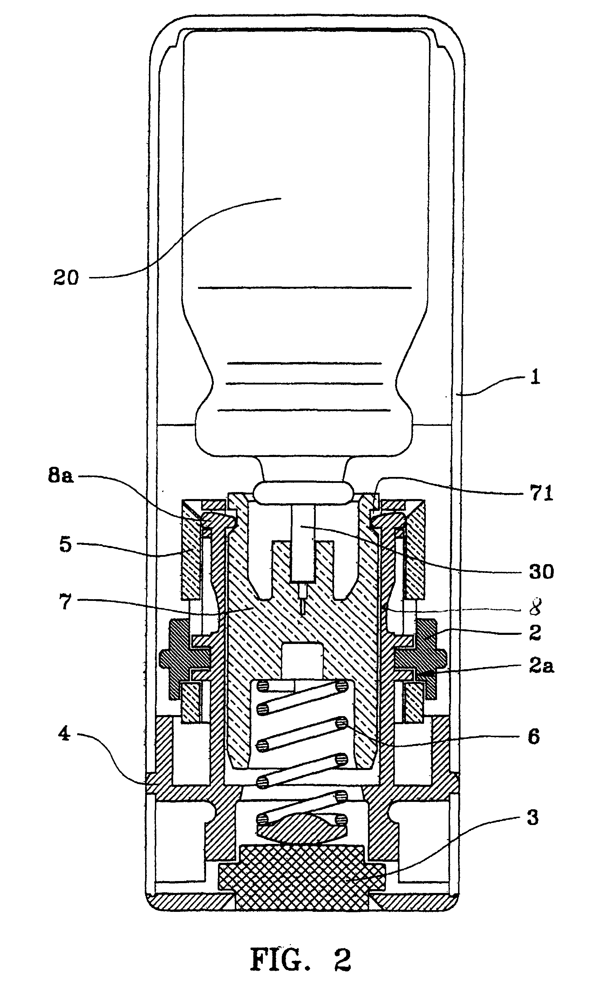

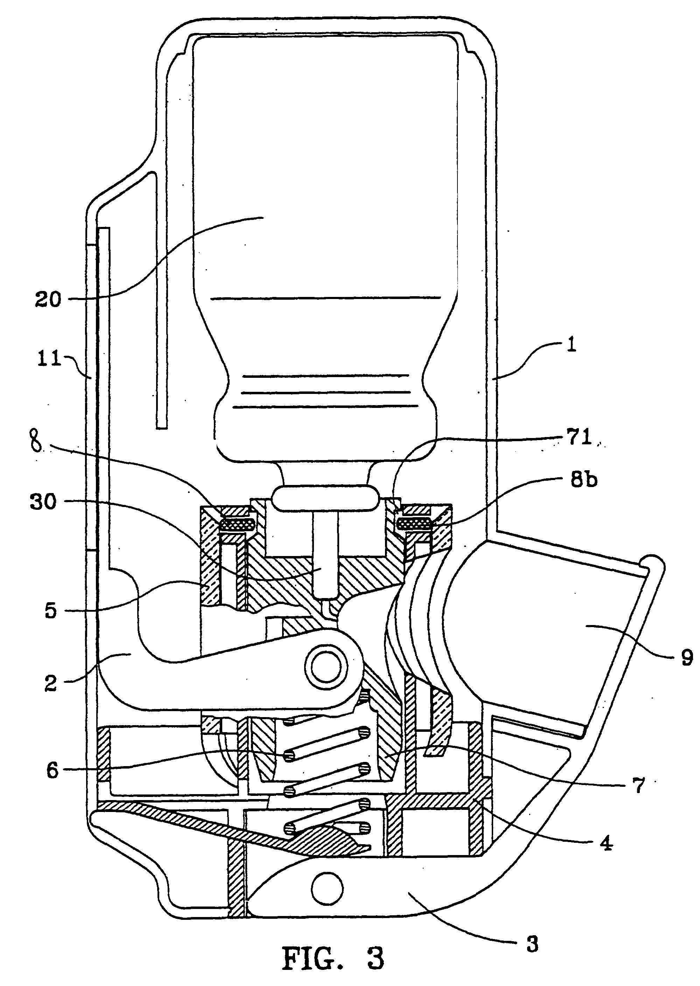

Le dispositif de déclenchement comporte en outre un élément de retenue 8 qui est adapté à coopérer avec l'organe d'actionnement 7. Cet élément de retenue 8 est mobile entre une position de retenue, dans laquelle il retient l'organe d'actionnement 7 dans sa position de non-actionnement, telle que représentée sur les figures 2 et 3, et une position de libération, dans laquelle ledit organe d'actionnement 7 peut se déplacer en direction de sa position d'actionnement, comme représenté sur la figure 5. Cet élément de retenue 8 est de préférence élastiquement déformable entre sa position de retenue et sa position de libération et est sollicité vers sa position de libération par l'organe d'actionnement 7 lorsque lui-même est sollicité vers sa position d'actionnement. L'élément de retenue 8 peut être réalisé sous la forme d'un anneau fendu 8b, comme représenté sur la figure 3, ou sous la forme d'une ou plusieurs pattes flexibles élastiques 8a comme représenté sur la figure 2. Avantageusement, l'élément de retenue 8 est logé dans une rainure 71 ménagée dans l'organe d'actionnement 7, cette rainure 71 ayant de préférence une paroi latérale oblique, ce qui a pour effet d'exercer une force radiale sur l'élément de retenue 8 lorsque l'organe d'actionnement 7 est soumis à une force axiale en étant sollicité vers sa position d'actionnement.

Selon l'invention, l'élément de came 2 dans sa position de stockage, est adapté à bloquer l'élément de retenue 8 dans sa position de retenue. Lorsque l'élément de came 2 est déplacé dans sa position d'inhalation, ledit élément de retenue 8 peut se déplacer vers sa position de libération, de sorte que l'organe d'actionnement 7 peut alors se déplacer vers sa position d'actionnement pour actionner la pompe ou valve doseuse 30.

De préférence, le dispositif de déclenchement comporte un organe de blocage 5 qui coopère d'une part avec l'élément de retenue 8 pour le bloquer dans sa position de retenue tant que l'élément de came 2 est dans sa position de stockage, et d'autre part avec l'élément de came 2, de sorte qu'un déplacement de l'élément de came 2 vers sa position d'inhalation agit sur l'organe de blocage 5 pour le déplacer de sa position de blocage vers une position de déblocage de l'élément de retenue. Avantageusement, cet organe de blocage est réalisé sous la forme d'un manchon 5 pouvant coulisser autour de l'organe de retenue 8 pour empêcher en position de blocage la déformation radiale de celui-ci vers sa position de libération. De préférence, ledit manchon 5 comporte une première partie cylindrique qui coopère en position de blocage avec l'élément de retenue 8, et une seconde partie de plus grand diamètre qui coopère avec l'élément de retenue 8 en position de déblocage pour lui permettre de se déplacer par déformation élastique vers sa position de libération. Avantageusement, cette seconde partie du manchon 5 est réalisée de manière conique, cette forme particulière pouvant être avantageuse après l'inhalation d'une dose de produit, pour ramener le dispositif de déclenchement par l'inhalation dans sa position de repos initiale comme cela sera expliqué ci-après.

Avantageusement, le dispositif de déclenchement par inhalation comporte un élément de structure 4 qui est fixé au boîtier 1, et sur lequel est monté pivotant l'organe de commande 3 ainsi que l'élément de came 2, comme visible sur la figure 2. Selon la variante de réalisation qui est représentée sur la figure 2, l'élément de retenue 8 peut être réalisé sous la forme d'une ou de plusieurs pattes flexibles élastiques 8a, celles-ci étant avantageusement réalisées d'une pièce avec ledit élément de structure 4. Eventuellement, l'élément de structure 4 peut comporter une languette flexible disposée entre l'organe de commande 3 et le ressort 6 pour faciliter la coopération entre ces deux éléments.

Le fonctionnement du dispositif selon l'invention est le suivant.

Avant que l'utilisateur ne souhaite utiliser le dispositif pour s'administrer une dose de produit, ledit dispositif est dans la position de repos représentée sur les figures 1, 2 et 3, dans laquelle l'organe de commande 3 est en position de repos, obturant l'embout buccal 9, l'organe d'actionnement 7 est en position de non-actionnement et n'est pas sollicité en direction de sa position d'actionnement par le ressort 6 qui n'est pas comprimé, l'élément de retenue 8 étant en position de retenue et l'organe de blocage 5 en position de blocage alors que l'élément de came 2 est en position de stockage obturant l'ouverture 11 du boîtier. De préférence, l'élément de retenue 8 n'est pas vers sa position de libération tant que l'organe d'actionnement 7 n'est pas sollicité vers sa position d'actionnement.

Lorsque l'utilisateur souhaite utiliser le dispositif, il déplace l'élément de commande 3 vers sa position de commande, représentée sur la figure 4. Ceci a pour effet d'ouvrir l'embout buccal 9 d'une part, et d'autre part, de comprimer le ressort 6 qui exerce alors une force axiale sur l'organe d'actionnement 7 pour solliciter ce dernier vers sa position d'actionnement. L'organe d'actionnement 7 est toutefois retenu dans sa position de non-actionnement par l'élément de retenue 8 qui coopère avec la rainure 71 de l'organe d'actionnement 7. La paroi latérale oblique de cette rainure 71 exerce une force radiale sur l'élément de retenue 8 pour solliciter ce dernier vers sa position de libération. Toutefois, l'élément de retenue 8 est bloqué dans sa position de retenue par le manchon de blocage 5 qui est en position de blocage et qui empêche toute déformation de l'élément de retenue 8. Le dispositif est donc en position d'attente.

Lorsque l'utilisateur inhale à travers l'embout buccal 9, il crée une dépression à l'intérieur du boîtier 1 qui a pour effet de faire pivoter l'élément de came 2 de sa position de stockage, représentée sur la figure 4, vers sa position d'inhalation, représentée sur la figure 5. En particulier, la partie de volet de l'élément de came 2 est décollée de l'ouverture 11 dans le boîtier 1 pour équilibrer la dépression créée à l'intérieur du boîtier. Ce déplacement de l'élément de came 2 de sa position de stockage vers sa position d'inhalation a pour effet de faire coulisser le manchon de blocage 5 axialement par rapport à l'élément de retenue 8. Avantageusement, une partie de came 2a de l'élément de came 2 traverse le manchon 5 et tourne par rapport à celui-ci lorsque l'élément de came 2 se déplace ves sa position d'inhalation, de sorte que ledit manchon 5 se déplace en synchronisation avec ledit élément de came 2. L'élément de retenue 8, sous l'effet de la force exercée sur lui par l'organe d'actionnement 7, se déforme alors vers sa position de libération, permettant ainsi à l'organe d'actionnement 7 d'être propulsé vers sa position d'actionnement par le ressort 6 comprimé qui agit sur lui. La dose de produit est alors expulsée à travers la pompe ou valve doseuse 30 en direction de l'embout buccal 9, pour être inhalée par l'utilisateur de manière synchronisée avec son inhalation.

En fin d'inhalation, l'utilisateur ramène l'élément de commande 3 dans sa position de repos dans laquelle il obture l'embout buccal 9. Ceci permet au ressort 6 de se relâcher et d'être ramenée vers sa position de repos, de sorte que l'organe d'actionnement 7 revient vers sa position de non-actionnement avec la rainure 71 en regard de l'élément de retenue 8. Celui-ci revient alors dans sa position de retenue, soit par sa propre élasticité, soit en étant légèrement aidé par la partie conique de l'organe de blocage 5 qui, dont le déplacement axial de sa position de déblocage vers sa position de blocage, exerce une force radiale vers l'intérieur sur ledit élément de retenue 8. L'élément de retenue 8 revient donc dans sa position de retenue, le manchon de blocage 5 dans sa position de blocage et l'élément de came 2 dans sa position de stockage, de sorte que le dispositif est prêt pour une utilisation ultérieure.

La présente invention permet donc de fournir un dispositif de distribution de produit fluide déclenché par l'inhalation, dans lequel le dispositif de déclenchement par inhalation est particulièrement simple et donc peu coûteux à fabriquer et à réaliser car il comporte un nombre de pièces constitutives minimal. Ainsi, dans son mode de réalisation préféré, qui est celui représenté sur les dessins, le dispositif de déclenchement comporte six ou sept pièces, à savoir l'élément de commande 3, l'élément de structure 4, le ressort 6, l'organe d'actionnement 7, l'élément de retenue 8 s'il n'est pas réalisé d'une pièce avec l'élément de structure 4, l'organe de blocage 5 et l'élément de came 2. Il pourrait même être envisagé de réaliser le dispositif de déclenchement avec un nombre de pièces encore diminué, par exemple en réalisant l'élément de came 2 de telle sorte qu'il incorpore lui-même l'organe de blocage 5. La présente invention permet donc de fournir un dispositif de déclenchement par inhalation qui est fiable tout en étant simple et peu coûteux.

## Revendications

1. Dispositif de distribution de produit fluide déclenché par l'inhalation, comportant un réservoir de produit (20) contenant un gaz propulseur, une pompe ou valve doseuse (30) pour distribuer sélectivement le produit à travers un orifice de distribution (9), et un dispositif de déclenchement commandé par l'inhalation, dispositif de déclenchement comportant :
- un élément de commande (3) mobile entre une position de repos, dans laquelle il obture l'orifice de distribution (9), et une position de commande, ledit élément de commande (3) étant déplacé par l'utilisateur,
- un organe d'actionnement (7), mobile entre une position de non-actionnement, dans laquelle la pompe ou valve doseuse (30) n'est pas actionnée, et une position d'actionnement, dans laquelle la pompe ou valve doseuse (30) est actionnée, ledit organe d'actionnement (7) étant sollicité vers sa position d'actionnement lorsque l'élément de commande (3) est dans sa position de commande,
- un élément de retenue (8), mobile entre une position de retenue, dans laquelle il coopère avec l'organe d'actionnement (7) pour le retenir dans sa position de non-actionnement, et une position de libération, dans laquelle ledit organe d'actionnement (7) se déplace dans sa position d'actionnement, et
- un élément de came (2), mobile entre une position de stockage, dans laquelle ledit élément de retenue (8) est bloqué en position de retenue, et une position d'inhalation, dans laquelle ledit élément de retenue se déplace dans sa position de libération pour libérer l'organe d'actionnement, ledit élément de came (2) étant sollicité vers sa position de stockage et étant déplacé dans sa position d'inhalation lors de l'inhalation par l'utilisateur du dispositif,
**caractérisé en ce que** l'élément de retenue (8) est élastiquement déformable entre sa position de retenue et sa position de libération, l'élément de retenue (8) étant sollicité vers sa position de libération par l'organe d'actionnement (7) lorsque l'élément de commande (3) est dans sa position de commande.

2. Dispositif selon la revendication 1, comportant en outre un organe de blocage (5) coopérant d'une part avec l'élément de retenue (8) et d'autre part avec l'élément de came (2), ledit organe de blocage (5) étant mobile entre une position de blocage, dans laquelle il bloque ledit élément de retenue (8) en (5) étant en position de blocage lorsque ledit élément de came (2) est dans sa position de stockage, et en position de déblocage, lorsque ledit élément de came (2) est dans sa position d'inhalation.

3. Dispositif selon la revendication 1 ou 2, dans lequel le déplacement de l'élément de commande (3) vers sa position de commande comprime un ressort (6) qui sollicite l'organe d'actionnement (7) vers sa position d'actionnement.

4. Dispositif selon la revendication 3, dans lequel le ressort (6) est disposé entre l'élément de commande (3) et l'organe d'actionnement (7).

5. Dispositif selon l'une quelconque des revendications précédentes, comportant un boîtier (1) recevant le réservoir (20), la pompe ou valve doseuse (30) et le dispositif de déclenchement, le boîtier (1) comportant en outre l'orifice de distribution (9), tel qu'un embout buccal, à travers lequel l'utilisateur inhale, et une ouverture (11), obturée par l'élément de came (2) en position de stockage.

6. Dispositif selon la revendication 5, dans lequel ledit élément de came (2) est monté pivotant dans le boîtier (1), l'inhalation de l'utilisateur créant une dépression dans le boîtier (1) qui fait pivoter l'élément de came (2) vers sa position d'inhalation, dans laquelle il n'obture plus l'ouverture (11) du boîtier (1).

7. Dispositif selon la revendication 5 ou 6, dans lequel l'élément de commande (3) est monté pivotant sur le boîtier (1).

8. Dispositif selon l'une quelconque des revendications précédentes, dans lequel l'élément de retenue (8) est logé dans une rainure (71) de l'organe d'actionnement (7), ladite rainure (71) ayant une paroi latérale oblique, qui, lorsque l'organe d'actionnement (7) est sollicité vers sa position d'actionnement, exerce une force sur l'élément de retenue (8) pour le solliciter vers sa position de libération.

9. Dispositif selon la revendication 2, dans lequel l'organe de blocage (5) est un manchon cylindrique monté coulissant autour de l'élément de retenue (8), ledit manchon (5) comportant une première partie coopérant en position de blocage avec l'élément de retenue (8) pour l'empêcher de se déplacer vers sa position de libération, et une seconde partie de plus grand diamètre coopérant en position de déblocage avec l'élément de retenue (8) en lui permettant de se déplacer vers sa position de libération.

10. Dispositif selon la revendication 9, dans lequel la seconde partie du manchon (5) est conique, de telle sorte que lorsque le manchon (5) est ramené dans sa position de blocage, la partie conique du manchon (5) coopère avec l'élément de retenue (8) pour le solliciter vers sa position de retenue.

11. Dispositif selon l'une quelconque des revendications 5 à 7, comportant en outre un élément de structure (4) fixé au boîtier (1), l'élément de came (2) et l'élément de commande (3) étant montés pivotants sur ledit élément de structure (4).

12. Dispositif selon l'une quelconque des revendications précédentes, dans lequel l'élément de retenue (8) est un anneau fendu déformable (8b).

13. Dispositif selon la revendication 11, dans lequel l'élément de retenue (8) comporte au moins une patte flexible déformable (8a) solidaire dudit élément de structure (4).

14. Dispositif selon la revendication 2, dans lequel, après distribution de la dose de produit par la pompe ou valve doseuse (30), l'élément de commande (3), lorsqu'il est ramené dans sa position de repos, ramène l'organe d'actionnement (7) dans sa position de non-actionnement, de sorte que l'élément de retenue (8) est ramené dans sa position de retenue, l'organe de blocage (5) est ramené dans sa position de blocage et l'élément de came (2) est ramené dans sa position de stockage.

## Patentansprüche

1. Vorrichtung zur Abgabe eines fluidförmigen Produktes, die durch Inhalation ausgelöst wird, mit einen Produktbehälter (20), der ein Treibgas enthält, einer Dosierpumpe oder einem Dosierventil (30) zum wahlweisen Abgeben des Produktes durch eine Abgabeöffnung (9) und einer Auslösevorrichtung, die durch Inhalation gesteuert wird, wobei die Auslösevorrichtung folgende Bestandteile umfasst:
- ein Steuerelement (3), das zwischen einer Ruhelage, in der es die Abgabeöffnung (9) verschließt und einer Steuerungsposition bewegbar ist, wobei das Steuerelement (3) durch den Verwender verschiebbar ist,
- ein Betätigungsorgan (7), das zwischen einer Nichtbetätigungsposition, in der die Dosierpumpe oder das Dosierventil (30) nicht betätigt wird, und einer Betätigungsposition bewegbar ist, in der die Dosierpumpe oder das Dosierventil (30) betätigt wird, wobei das Betätigungsorgan (7) in seine Betätigungsposition vorgespannt ist, wenn sich das Steuerelement (3) in seiner Steuerungsposition befindet,
- ein Rückhalteelement (8), das zwischen einer Rückhalteposition, in der es mit dem Betätigungsorgan (7) zusammenwirkt, um dieses in seiner Nichtbetätigungsposition zurückzuhalten, und einer Freigabeposition bewegbar ist, in der sich das Betätigungsorgan (7) in seine Betätigungsposition verschiebt, und
- ein Nockenelement (2), das zwischen einer Speicherposition, in der das Rückhalteelement (8) in der Rückhalteposition blockiert ist, und einer Inhalationsposition bewegbar ist, in der das Rückhalteelement sich in seine Freigabeposition verschiebt, um das Betätigungsorgan freizugeben, wobei das Nockenelement (2) zu seiner Speicherposition hin vorgespannt ist und in seine Inhalationsposition verschoben wird, wenn der Verwender der Vorrichtung inhaliert,
**dadurch gekennzeichnet, dass** das Rückhalteelement (8) in elastischer Weise zwischen seiner Rückhalteposition und seiner Freigabeposition verformbar ist, wobei das Rückhalteelement (8) zu seiner Freigabeposition hin durch das Betätigungsorgan (7) vorgespannt ist, wenn sich das Steuerelement (3) in seiner Steuerungsposition befindet.

2. Vorrichtung nach Anspruch 1, die weiterhin ein Blockierorgan (5) umfasst, das einerseits mit dem Rückhalteelement (8) und andererseits mit dem Nockenelement (2) zusammenwirkt, wobei das Blockierorgan (5) zwischen einer Blockierposition, in der es das Rückhalteelement (8) in seiner Rückhalteposition blockiert, und einer Deblockierposition bewegbar ist, in der das Rückhalteelement sich in seine Freigabeposition verschiebt, wobei das Blockierorgan (5) sich dann in seiner Blockierposition befindet, wenn sich das Nockenelement (2) in seiner Speicherposition befindet, und in seiner Deblockierposition, wenn sich das Nockenelement (2) in seiner Inhalationsposition befindet.

3. Vorrichtung nach Anspruch 1 oder 2, bei der die Verschiebung des Steuerelementes (3) zu seiner Steuerungsposition hin eine Feder (6) zusammendrückt, die das Betätigungsorgan (7) zu seiner Betätigungsposition hin vorspannt.

4. Vorrichtung nach Anspruch 3, bei der die Feder (6) zwischen dem Steuerelement (3) und dem Betätigungsorgan (7) angeordnet ist.

5. Vorrichtung nach einem der vorhergehenden Ansprüche, die ein Gehäuse (1) umfasst, welches den Behälter (20), die Dosierpumpe oder das Dosierventil (30) und die Auslösevorrichtung aufnimmt, wobei das Gehäuse (1) weiterhin die Abgabeöffnung (9) sowie einen Nasenansatz umfaßt, durch den hindurch der Verwender inhaliert, sowie eine Öffnung (11), die von dem Nockenelement (2) in der Speicherposition verschlossen ist.

6. Vorrichtung nach Anspruch 5, bei der das Nockenelement (2) in schwenkbarer Weise in dem Gehäuse (1) montiert ist, wobei das Inhalieren des Verwenders einen Unterdruck im Gehäuse (1) erzeugt, der das Nockenelement (2) in seine Inhalationsposition schwenken läßt, in der es die Öffnung (11) des Gehäuses (1) nicht mehr verschließt.

7. Vorrichtung nach Anspruch 5 oder 6, bei der das Steuerelement (3) an dem Gehäuse (1) in schwenkbarer Weise montiert ist.

8. Vorrichtung nach einem der vorhergehenden Ansprüche, bei der das Rückhalteelement (8) in einer Rille (71) des Betätigungsorgans (7) untergebracht ist, wobei die Rille (71) eine schräge Seitenwand besitzt, die dann, wenn das Betätigungsorgan (7) zu seiner Betätigungsposition hin vorgespannt ist, eine Kraft auf das Rückhalteelement (8) ausübt, um dieses zu seiner Freigabeposition hin vorzuspannen.

9. Vorrichtung nach Anspruch 2, bei der das Blockierorgan (5) eine Zylinderbuchse ist, die in verschiebbarer Weise um das Rückhalteelement (8) herum montiert ist, wobei die Buchse (5) einen ersten Teil aufweist, der in der Blockierposition mit dem Rückhalteelement (8) zusammenwirkt, um dieses daran zu hindern, sich zu seiner Freigabeposition hin zu verschieben, sowie einen zweiten Teil mit größerem Durchmesser, der in der Deblockierposition mit dem Rückhalteelement (8) in der Weise zusammenwirkt, dass er es ihm ermöglicht, sich zu seiner Freigabeposition hin zu verschieben.

10. Vorrichtung nach Anspruch 9, bei der der zweite Teil der Buchse (5) derart konisch ist, dass dann, wenn die Buchse (5) in ihre Blockierposition zurückgeholt wird, der konische Teil der Buchse (5) mit dem Rückhalteelement (8) zusammenwirkt, um dieses zu seiner Rückhalteposition hin vorzuspannen.

11. Vorrichtung nach einem der Ansprüche 5 bis 7, die weiterhin ein Strukturelement (4) umfaßt, das am Gehäuse (1) befestigt ist, wobei das Nockenelement (2) und das Steuerelement (3) in schwenkbarer Weise an dem Strukturelement (4) montiert sind.

12. Vorrichtung nach einem der vorhergehenden Ansprüche, bei der das Rückhalteelement (8) ein verformbarer, geschlitzter Ring (8b) ist.

13. Vorrichtung nach Anspruch 11, bei der das Rückhalteelement (8) wenigstens einen flexiblen, verformbaren Stift (8a) umfasst, der mit dem Strukturelement (4) fest verbunden ist.

14. Vorrichtung nach Anspruch 2, bei der nach der Abgabe der Dosis durch die Dosierpumpe oder das Dosierventil (30) das Steuerelement (3) dann, wenn es in seine Ruhelage zurückgeholt wird, das Betätigungsorgan (7) in seine Nichtbetätigungsposition derart zurückholt, dass dann, wenn das Rückhalteelement (8) in seine Rückhalteposition zurückgeholt wird, das Blockierorgan (5) in seine Blockierposition und das Nockenelement (2) in seine Speicherposition zurückgeholt wird.

## Claims

1. Dispensing device for a fluid product activated by inhaling, which comprises a product reservoir (20) containing a propellant gas, a pump or dosing valve (30) to selectively dispense the product through a dispensing orifice (9), and an activating device controlled by inhaling, an activating device comprising:
- a control element (3) mobile between an inoperative position in which it blocks the dispensing orifice (9) and a control position, said control element (3) being displaced by the user,
- an actuating member (7), mobile between a non-actuating position, in which the pump or dosing valve (30) is not actuated and an actuating position in which the pump or dosing valve (30) is actuated, said actuating member (7) being urged towards its actuating position when the control element (3) is in its control position,
- a retaining element (8), mobile between a retaining position in which it co-operates with the actuating element (7) to retain it in its non-actuating position and a releasing position, in which said actuating member (7) moves into its actuating position, and
- a cam element (2), mobile between a storing position in which said retaining element (8) is locked in its retaining position, and an inhaling position, in which said retaining element moves into its releasing position to release said actuating element, said cam element (2) being urged towards its storing position and being moved into its inhaling position when the user of the device inhales,
**characterized in that** the retaining element (8) is elastically deformable between its retaining position and its releasing position, the retaining element (8) being urged towards its releasing position by the actuating member (7) when the control element (3) is in its control position.

2. Device according to Claim 1, additionally comprising a locking member (5) that co-operates, on the one hand with the retaining element (8) and on the other hand, with the cam element (2), said locking member (5) being mobile between a locking position in which it locks said retaining element (8) in its retaining position, and an unlocking position, in which said retaining element moves into its releasing position, said locking member (5) being in the locking position when said cam element (2) is in its storing position, and in the unlocking position, when said cam element (2) is in its inhaling position.

3. Device according to Claim 1 or 2, in which movement of the control element (3) towards its control position compresses a spring (6) which urges the actuating member (7) towards its actuating position.

4. Device according to Claim 3, in which the spring (6) is arranged between the control element (3) and the actuating member (7).

5. Device according to any one of the preceding Claims comprising a casing (1) that receives the reservoir (20), the pump or dosing valve (30) and the activating device, the casing (1) additionally comprising the dispensing orifice (9), such as an end mouth-piece, through which the user inhales, and an opening (11) blocked off by the cam element (2) in the storing position.

6. Device according to Claim 5, in which said cam element (2) is pivotably mounted in the casing (1) inhalation by the user creating a pressure drop in the casing (1) which causes the cam element (2) to pivot towards its inhaling position, in which it no longer blocks the opening (11) of the casing (1).

7. Device according to Claim 5 or 6, in which the control element (3) is pivotably mounted on the casing (1).

8. Device according to any one of the preceding Claims, in which the retaining element (8) is housed in a groove (71) of the actuating member (7), said groove (71) having a slanting side wall which, when the actuating member (7) is urged towards its actuating position, exerts a force on the retaining element (8) to urge it towards its releasing position.

9. Device according to Claim 2, in which the locking member (5) is a cylindrical sleeve slidably mounted around the retaining element (8), said sleeve (5) comprising a first part that in the locking position, co-operates with the retaining element (8) to prevent it being moved towards its releasing position and a second part with a larger diameter, which, in the unlocking position, co-operates with the retaining element (8) and enables it to be moved towards its releasing position.

10. Device according to Claim 9, in which the second part of the sleeve (5) is conical so that when the sleeve (5) is brought into its locking position, the conical part of the sleeve (5) co-operates with the retaining element (8) to urge it towards its retaining position.

11. Device according to any one of Claims 5 to 7, additionally comprising a structural element (4) fixed to the casing (1), the cam element (2) and the control element (3) being pivotably mounted on said structural element (4).

12. Device according to any one of the preceding Claims in which the retaining element (8) is a deformable split ring (8b).

13. Device according to Claim 11, in which the retaining element (8) comprises at least one flexible deformable foot (8a) integral with said structural element (4).

14. Device according to Claim 2, in which, after dispensing the dose of product through the pump or dosing valve (30), the control element (3), when it is returned to its inoperative position, returns the actuating member (7) to its non-actuating position, so that the retaining element (8) is returned to its retaining position, the locking member (5) is returned to its locking position and the cam element (2) is returned to its storing position.
